# EUROPEAN PATENT SPECIFICATION

(11) **EP 2 185 129 B1**
(45) Date of publication and mention of the grant of the patent: **21.10.2015**
(21) Application number: 08789057.0
(22) Date of filing: 04.08.2008
(51) Int. Cl.: A61K 9/00, A23K 1/00

(54) **PELLET FOR ANIMALS AND METHOD FOR ITS PRODUCTION**
PELLET FÜR TIERE UND VERFAHREN ZU SEINER HERSTELLUNG
GRANULÉS POUR ANIMAUX ET LEUR PROCÉDÉ DE FABRICATION

(30) Priority: 08.08.2007 IT PD20070274
(43) Date of publication of application: 19.05.2010
(73) Proprietor: Sanypet S.p.a., 35023 Bagnoli di Sopra (PD) (IT)
(72) Inventor: GUIDETTI, Gianandrea, I-42100 Reggio Emilia (IT); CANELLO, Sergio, 35037 Villa di Teolo (PD) (IT)
(74) Representative: Gallo, Luca
(86) International application number: PCT/IB2008/002107
(87) International publication number: WO 2009/019593

(56) References cited:
- EP-A- 0 036 345
- EP-A- 0 149 140
- AU-B2- 621 282
- FR-A- 2 313 079
- GB-A- 1 290 811
- NL-C1- 1 003 855
- US-A- 3 466 370
- US-A- 4 001 411
- US-A- 4 495 177
- US-A- 5 994 395

## Description

### Field of application

The present invention concerns product for feeding animals and process for producing a product for animal according to the introduction of claims 1 and 8.

The invention falls within the animal food and care products industry, in particular for animals of the domestic type such as dogs and cats.

The pellet subject of the present invention shall be advantageously employed for administering drugs, supplements, vitamins, physiotherapy substances or substances sensitive to heat and/or oxidising/reducing substances and more generally for administering active ingredients for therapeutic, medical or prevention purposes.

### Prior art

As known in the field of veterinary, in particular for the care of domestic animals, of particular concern is the problem regarding administration of drugs or physiotherapy substances, together with the food products in a form capable of maintaining the properties and the activity of the therapeutic active ingredients intact.

Currently, for practical reasons, many drugs used for animal care are administered by introducing them directly into the food products.

As a matter fact, drugs, supplements and the like usually do not have a taste pleasant to animals and they would be easily detected and rejected if administered separately.

This method of administering drugs and supplements to animals by adding them to the food product is widely considered a consolidated method offering indisputable practical advantages.

However, such administration practice, raises problems regarding the correct dosage and preservation of the activity of the active ingredients inside the food products.

Food products for animals are usually obtained according to modern production process in form of pellets, which have proved to be particularly suitable for feeding various animal species and in particular for domestic species such as dogs and cats.

Pellets are obtained by means of hot extrusion industrial processes starting from multi-components which are dried and packaged once shaped into pellets.

More in detail, the extruded food products specifically prepared according to the nutritional needs of the type of animal they are intended for, generally provide for suitably mixing together, in a dosed manner, one or more cereals or their by-products, flours, protein concentrates of animal or vegetable origin, fats.

The mixture of the components thus obtained is introduced into an extrusion chamber where it is subjected to heating, generally in the range of 85°C to 130°C, before being forced by the action of one or two worm screws to pass through the holes of a die arranged on the head of the extruder. The mixture exiting from the die is cut by blades into the shape of the pellets desired.

Once dried, the product is free of microbial contaminations and particularly digestible.

Lastly, in order to increase the tastefulness of the pellets it is possible to subject the extruded granules to fat-covering processes and/or spraying with products of animal origin such as for example meat, giblets, fats.

Should the pellets be used as vehicles for administering drugs it should be borne in mind that the production processes indicated above subject the medical substances, vitamins, phytotherapy extracts, and active ingredients contained therein to considerable thermal stress.

Most of these active ingredients are partially inactivated by the heat losing their active antioxidant function. In particular, many vitamins are extremely sensitive to the temperature, that is they are entirely thermolabile even in the presence of minimum thermal excursions.

Furthermore, during the production process, the drugs come into contact with mixtures having a considerable percentage of water or moisture which facilitate the oxidation and functional deterioration of most of the active ingredients.

Lastly, thermal stresses and moisture linked to the current production processes quickly deteriorate the activity characteristics of the drug principles added to the pellets.

Consequently, most of the pellets available in the market proved to be inappropriate as vehicles suitable for administration of drug substances for animals.

In order to overcome this drawback, special dual component pellets obtained through co-extrusion and provided with an external shell coating to protect a core containing the active ingredients of the drug or sensitive substances, have been studied and introduced into the market.

More in detail, for example the US 6,117,477 patent tackles the problem of producing food for animals capable of reducing the use of water and allowing to add functional, nutritional and pharmaceutical components protected against heat, light and oxygen for example, having anti-inflammatory functions, curing functions for problems regarding the respiratory system, to improve the immune response, with antioxidation functions, with parasite control functions, with antibiotic functions etc. This patent described a dual component product provided by means of co-extrusion wherein the internal component is obtained without passing through aqueous phases, has a percentage of moisture lesser than 15% by weight and contains a mixture of lipids and solid ingredients forming a cream; while the second component covers the first component completely, such product has a percentage of moisture lesser than 20% by weight and it contains at least one ingredient containing a carbohydrate, a fat, protein or their combination.

The US 6,254,910 patent proposes providing a food product vehicle for administering to animals various nutritional, functional or pharmaceutical additives sensitive to heat, light or oxygen, to prevent them from being altered or destroyed by processes including heat or acid conditions. For this purpose, the patent provides for the production of an edible dual component product obtained through co-extrusion of a first component obtained without aqueous phases, such product having a percentage of moisture lesser than 15% by weight, containing a mixture of lipids and solid ingredients forming a cream, and containing an ingredient with an unstable process or using ingredients sensitive to heat, light or oxygen; and a second component with a percentage of moisture lesser than 20% by weight and containing at least one ingredient containing a carbohydrate, a fat, protein or their combination and completely coating the first component in form of cream and thus maintaining the vitality of the ingredient sensitive to heat, light, oxygen or with unstable process.

Analogously, the US 6,506,401 patent provides for the production of an edible product for animals useable for treating the digestion of hairballs in a form comprising an emulsion with malt syrup with an aqueous and an oil phase; and a second covering edible component different from the past and applied to coat in order to encapsulate the formulation.

Though they allowed enhancing the effectiveness of the drugs partially preserving the activity of their active ingredients, the abovementioned co-extrusion processes did not allow achieving particularly satisfactory results.

As a matter of fact, the extrusion process requires providing the heart-shaped product, containing the active ingredients, in form of cream to be extruded and thus with a moisture, though low, incompatible for a complete preservation of the properties of the active ingredients of the drugs.

Furthermore, the co-extrusion process still requires heating the external shell which transmits - by conduction - part of the heat to the heart inserted mixture. Such heat is enough to inhibit the activity of most curative principles contained in drugs added the heart-shaped component.

Furthermore, the co-extrusion process in practice proved quite expensive and complicated to manufacture.

Document. EP 0149140 discloses a bolus for veterinary administration comprising corn starch and magnesium stearate. The bolus is prepared by direct compression and also comprises lactose. The bolus may also be added to the animal feed or drinking water.

### Presentation of the invention

In this situation, the problem on which the present invention is based is that of providing a product for feeding animals comprising pellets for therapeutic purposes capable preserving the functional activity characteristics of drugs, vitamins, supplements, phytotherapy substances and active ingredients in general for therapeutic purposes, added therein.

Another object of the present invention is that of providing product for feeding animals comprising pellets for therapeutic purposes which, though containing drugs, supplements, phytotherapy substances and active ingredients in general for therapeutic purposes, are tasty for the animals.

Another object of the present invention is providing product for feeding animals comprising pellets for therapeutic purposes containing drugs, supplements, phytotherapy substances and active ingredients for therapeutic purposes in general, capable of being mixed in doses or quantities of other pellets for feeding purposes remaining uniformly distributed therein.

Another object of the present invention is that of providing product for feeding animals comprising pellets for therapeutic purposes containing drugs, supplements, phytotherapy substances and active ingredients in general for therapeutic purposes, capable of being easily recognizable among doses or quantities of other pellets for feeding purposes.

Another object of the present invention is that of providing a process for producing a product for feeding animals comprising pellets for therapeutic purposes capable of ensuring preservation of chemical/physical conditions which do not damage, deteriorate or jeopardise the functionality of the active ingredients contained in the same pellets as a finished product.

These and other objects are all attained by means of the pellet for animals according to the annexed claims.

The technical characteristics of the invention, according to the abovementioned objects, are clearly observable from the content of the claims below and the advantages of the same shall be clearer in the detailed description hereinafter, provided with reference to an embodiment strictly for exemplifying and non-limited purposes.

### Detailed description

Hereinafter, the term pellet is generally used to indicate any agglomeration of ingredients in compact form, of size and hardness susceptible to allow its use even in a per se traditional manner and entirely similar to the use provided for the traditional pellets commonly used in the normal feeding animals, especially domestic animals such as dogs and cats, and well known to a man skilled in the art. Therefore, the pellet in question can be otherwise intended as a tablet, or still as a biscuit or granule, without for this reason departing from the scope of prevention of the present patent.

The pellet for therapeutic purposes is intended to be mixed uniformly in preset percentages and in a recognizable manner inside larger quantities of pellets intended for feeding animals.

According to a first important characteristic of the present invention the pellet for therapeutic purposes comprises a percentage of moisture lesser than 6% upon production.

Such characteristic allows maintaining the characteristics of the functional activity of drugs, vitamins, supplements, phytotherapy substances and active ingredients intact, in general for therapeutic purposes, added to the pellet and better specified hereinafter, which would otherwise be deteriorated by the action of water and oxygen held therein.

The pellet for therapeutic purposes is obtained starting from a mixture of ingredients in the form of powder, such ingredients being cold-compacted by means of simple pressing without adding liquids and thus without any exsiccation phase.

According to a second important characteristic of the invention, the ingredients comprise: a thickener component for compacting the ingredients together as indicated above by means of cold and dry compression; at least one hydrophilic mineral for absorbing the moisture from the other components of the tablet and facilitate the separation of the mixture compacted in form of pellet by the press; one or more therapeutic substances for curing animals with thermolabile active ingredients.

The therapeutic substance to be added to the pellet shall be selected, separately or combined with other substances depending on the curative purpose intended to be achieved as better specified hereinafter.

Though containing carbohydrates and other substances for feeding purposes, the pellet subject of the present invention is not solely intended for feeding animals but substantially to be a vehicle for administering therapeutic substances.

According to another characteristic of the present invention the abovementioned pellets for therapeutic purposes are mixed in the amounts of 5-15 % among pellets for feeding animals having the double therapeutic and feeding function.

The mixture of powder ingredients of the pellets for therapeutic purposes also comprises carob flour in a quantity comprised in the range of 5-25 %.

Pellets for feeding purposes are obtained by means of extrusion starting from a mixture of components introduced into an extrusion chamber and forced by the action of at least one worm screw, passing through the holes of a die arranged at the head of the extruder. The mixture exiting from the die is cut by blades in the desired form of pellets.

Lastly, the extruded granules are subjected to fat coating and/or spraying processes using products of animal or plant origin such as for example meat, giblets, fats (the so-called "digest" in the specialised technical language).

The pellets for therapeutic purposes are not subjected to the phases of fat coating and/or spraying but - having lipophilic characteristics - they absorb fat appetising substances by simple contact with the pellets for feeding purposes. In this manner, part of the appetising substances mainly made up of concentrated hydrolysed proteins extracted from chicken liver, giblets, substances particularly tasty for dogs and cats, are transferred onto the surface of the pellets for therapeutic purposes making also the latter quite appetising.

Given that no hot phase is used so as to allow starch to reticulate compacting the ingredients, it is necessary to add one or more thickening substances capable of performing the task of compacting ingredients, even in a cold manner, by means of simple compression.

The thickening substance shall preferably be selected from one or more ingredients selected from among: carob flour, calcium dibasic phosphate, maize starch, maltodextrine.

Preferably, a measured combination of these ingredients can be selected and in particular 40-55% of calcium dibasic phosphate, 5-25% of carob flour, 25 - 50 % of maize starch.

The considerable amount of calcium phosphate is justified by the aim of providing the required amount of salts for the animals as well as the fact that it is a good compacting agent. Such amount can be reduced in case the pellet is mixed with other pellets, for feeding purposes, already rich with such salts.

Advantageously, it was discovered that the carob flour, added to the mixture at a quantity comprised in the range of 5-25 % and preferably at 10%, serves the double purpose of being highly compacting by means of compression and being considerably tasty for the animals. Furthermore, the carob flour alongside the other components, when the pellet is added to and mixed with pellets for the traditional type of feeding, that is treated externally with appetising fats, leads the pellet to absorbing part of this part and thus be more tasty for the animals.

Surprisingly, the absorption of this part confers to the pellet - due to the carob flour contained therein - a very dark colour (dark brown) making the pellets subject of the present invention easily recognizable inside a mixture with other pellets for feeding purposes. This is a clear advantage given that it allows a visual control in a manner such that the pellets subject of the present invention for curative purposes are always administered to the animal substantially at the same doses during the normal administration of food. Otherwise, it allows increasing or reducing the daily dose of curative pellets intended to be administered to the animal by simply picking them up from the mixture. Furthermore, such recognizability is obtained in an entirely natural manner without requiring use of synthetic colorants.

As previously considered, according to a further characteristic of the present invention, the pellet comprises one or more hydrophilic minerals for absorbing the moisture from the other components of the tablet and facilitate the separation of the mixture compacted into pellets by the press.

Preferably, such mineral is magnesium stearate or silica which, by absorbing the moisture, make the other substances more dry preventing the active ingredients from oxidising upon contact with the oxygen of the water and avoiding complications of the bacteria type, encouraged by the moisture.

The therapeutic substance is selected separately or in combination, preferably in the family of products comprising: vitamin C, lysozyme, bioflavonoids, shark cartilage, contoutine, glucosamine, propolis, Echinacea, Astaxanthin, Papaya, Aloe, Ginseng, Pineapple, Rosa Canina, Papaya, Resveratrol, Carnetin, Resveratrol, and other phytotherapy substances, pharmaceutical ingredients or principles available in the market having stability problems under oxygen or heat.

The vitamins normally added to the pellets, under cold conditions, when coating with fat, shall also be advantageously added in the formulation of the pellet, for therapeutic purposes, subject of the present invention.

According to the first embodiment of the pellet subject of the present invention provided for is a pellet for dental hygiene and for protecting the vegetative system and the upper respiratory tract of dogs and cats preferably within a complete product providing for the use of the abovementioned pellet alongside pellets for feeding purposes.

According to the latest statistics, most dogs and cats, especially the small and medium sized ones, suffer pathologies regarding the oral cavity. Bad breath, tartar and gingivitis are common, very disturbing and, above all, they strongly influence the relationship with the owner of the animal.

Advantageously used in the therapeutic pellet are natural active ingredients such as Salvia, Thyme, Propolis, Vitamin C, Bioflavonoids, Lysozyme, combined into feeding pellets in particular obtained through percentages of sea fish (omega-3 and omega-6 fatty acids , extremely high protein value and high digestibility) and meat of organic origin in order to obtain the correct amount of protein.

Another product intended for curing joint problems provides for use of active ingredients such as chondroitin, glucosamine or shark cartilage, such substances being extremely thermolabile.

Also such therapeutic pellet is advantageously added to pellets for feeding purposes.

The present invention allows providing a diet efficient at both preventing various diseases by means of simple administration of pellets for feeding purposes, suitably completed with the pellets subject of the present invention.

The food product obtained form the combination of the two pellets is also an object of the present invention, and allows treating, in a practical and effective manner, many diseases caused by immunity deficiencies or infections. Many studies show that the immunity system is the system most vulnerable to the presence of various types environmental and food contaminations. As a consequence, the same system reacts, either in an excessive manner (allergies, autoimmune diseases), or in an insufficient manner (greater vulnerability to cancers and infective diseases such as, for example, Filiaris, Rickettsiosis, Lyme disease, Leishmaniosis).

Therefore, according to a further embodiment of the present invention, the active ingredients of the natural components (Echinacea, Astaxanthin, Papaya, Aloe) are added to the pellet for therapeutic purposes and they allow providing a complete product in particular in combination with pellets for feeding purposes comprising sea fish (omega-3 fatty acids, very high protein value and high digestibility) sunflower oil (slow tendency to oxidation). Such product is capable of offering a fundamental contribution both for preventing such diseases and during the specific therapy.

Further active ingredients that can be used in the pellets subject of the invention are Ginseng, Pineapple, Rosa Canina, Papaya, Resveratrol, Carnetin and Resveratrol selected by specialised chemists and veterinarians, and present in many formulations.

Considered more in detail hereinafter are some important therapeutic substances which, separately or combined, can be included in the formulation of the pellet subject of the present invention.

For some of them, no specific study was performed aimed at highlighting the degree of reduction of the activity of the active ingredients therein upon heating.

In its activity and due to its characteristics, the shark cartilage helps preventing inflammatory phenomena and reinforcing the immune defences. Shark cartilage is naturally rich in calcium, phosphorous, proteins, mucopolysaccharides, vitamins and collagen.

Chondroitin sulphate, jaluronic acid and glucosamine - present naturally - help regenerating the cartilaginous tissues, enhancing the health conditions of all the joints at the same time. Used in the present pellet, is a powder obtained through enzymatic hydrolysis of the shark skeleton.

The biological activity of the shark cartilage is particularly thermolabile, and it has been the subject of various researches which have confirmed that it is capable of:
acting as an effective natural anti-inflammatory;
accelerating the cicatrisation processes and contributing to the reinforcement and the recovery of the bone tissues, stimulating the cellular regeneration processes;
enhancing the production of antibodies by the animal organism, improving the immune response;
inducing a quick neuromuscular recovery.

The substance in question can also be indicated as a food adjuvant for therapies intended for various pathologies, among which: arthritis, dermatitis, psoriasis, herpes and ocular pathologies such as the cataract. Such substance helps the organism to prevent and fight the formation of new blood vessels in the tissues through its antiangiogenetic factor in a natural manner.

The Shark cartilage must contain 100% of dried and frozen cartilage, in a manner such to maintain the maximum bioactivity and effectiveness.

This is guaranteed by a process which provides for collecting the cartilage, controlling its quality, dehydrating under vacuum and pulverization strictly performed "cold": no boiling, cooking, enzymes or acids are used in the process.

Thus, this allows maintaining the maximum degree of effectiveness of the proteins and mucopolysaccharides which are not damaged in their activity when producing the pellets subject of the present invention.

The Echinacea is a plant of the Composite Tubiflore family, from whose roots useful extracts are obtained for curing diseases caused buy cold and for the prevention of minor respiratory pathologies (laryngitis, tracheitis, cough)

Taking Echinacea regularly may help preventing (especially during periods in which the organism is subjected to greater stress), shorten or soften the symptoms of diseases linked to cold, against attacks of flu, all the way to lip herpes.

It is also useful for prevention. A protective beneficent effect can also be observed regarding all the minor pathologies (coughs, sinusitis, colds).

Aloe Barbadensis is a succulent plant typical of the desert areas of Africa, Spain, Australia, Central America. Such plant is used for preventing and treating neoplastic diseases.

The gel is the most effective and non-toxic part of the plant.

The gel obtained from squeezing Aloe leaves proved to have anti-inflammatory activities. As a matter of fact, the gel is capable of reducing the degree of inflammation and stimulating the production of a new collagen. Some studies have shown that Aloe Vera Gel is capable of accelerating the natural healing process.

Aloe Vera Gel is capable of inhibiting the growth of pathogenic bacteria, especially at intestinal and skin level.

The bioflavonoids, present in considerable amounts especially in the citrus fruits we use, selectively act to maintain the natural integrity of the capillaries. From this point of view, their role is essential, given that the wellbeing and proper operation of the cells of the tissues depends on the nutrition they receive, which occurs through numerous capillaries present in the tissue.

The bioflavonoids serve for transporting hydrogen and for capturing circulating radicals, in a synergic manner with vitamin C. Then they act as important agents for detoxifying our organism.

All the vegetable parts of the plant contain important phytonutrients such as vitamins, mineral salts and carbohydrates, but the most important component for the phytotherapy activity is papain, an enzyme belonging to the same family as the pineapple bromelain.

Papain belongs to the group of proteinase, that is the enzymes that convert proteins into amino acids.

Through a conservative extraction process, obtained from the papaya fruit is a powder concentrated and standardised in proteolytic activity. The papaya extract is pale yellow in colour and soluble in water; it is stable but inactivated by heat, just like all enzymes.

The Papaya has a digestive, cicatrising, anti-inflammatory as well as antiseptic action and it is useful for stomatitis and gingivitis. The large amount of vitamins, mineral salts and carbohydrates guarantees various actions: regenerating, cytoenergetic and regulating.

Taking compounds with an antioxidant activity was associated to a reduction of risks regarding the occurrence of chronic diseases such as cardiovascular diseases and cancer. There is an ever-growing number research protocols for studying the effects of a specific antioxidant compound on health: Resveratrol.

Resveratrol is found in red wine and it seems to be one of the factors involved in the so-called "French paradox", that is the low incidence of cardiovascular diseases in France regardless of a diet rich in fat and red meat.

Taking this powerful antioxidant was associated to the reduction of risks related to contracting chronic diseases.

Though collection of further pharmacological and clinical regarding the activity of this compound is required, the preliminary results can already confirm the rationality of using Resveratrol as an ingredient with antioxidant properties capable of protecting the organism against oxidation stress, in particular regarding the cardiovascular system.

Regarding the immunity system, Vitamin C performs an action of enhancing the activity of the cells meant to defend the organism. Use of high doses of Vitamin C compensates its excessive loss during bouts of fever. Due to the intracellular transport it increases the bioavailability of the active ingredient (carrier effect). Furthermore, it has a protection action on the vascular system and it is good for activating defensive and immunity powers of the system.

Vitamin C performs a protection action, particularly in synergy with the bioflavonoids; thus, these two principles are essential to each other. Only the natural extracts guarantee the simultaneous presence of two active principles. Vitamin C facilitates the absorption of iron especially from vegetable sources. As a matter of fact, a proper daily supplementation with the vitamins, preserves the integrity of the animal and facilitates its wellbeing, in that it contributes to restore the delicate balance on which its metabolism is based.

Sage and Thyme are known for their antiseptic, antiedemigene and cicatrising properties. The phytocomplexes held therein, have values extremely rich in active ingredients with important or protective actions.

Sage and Thyme are efficient at fighting the excessive formation of catarrh in bronchial form. They are used as astringents, antiseptics, expectorants and mycolytics in the inflammations of the oropharyngeal cavity and they are also useful in asthmatic coughs.

Propolis is produced by bees and it is a brown coloured resin substance.

Advantageously, the density of the pellet for therapeutic purposes subject of the present invention is similar to the one of the pellet for feeding purposes to guarantee its uniformity of distribution inside the mass of the same pellets for feeding purposes, capable of resisting over time and not causing therapeutic pellets for example to heap at the bottom of the package making their administration to the animals non-uniform.

The uniformity in the distribution is facilitated by the shape of the therapeutic pellet subject of the invention, which is provided with corners preventing its easy movement inside a mass of other pellets for feeding purposes. In particular, preferable is a prism shape with substantially triangular upper and lower faces.

Preferably, natural preservatives such as tocopherols and extracts of rosemary are used in the feeding pellet, while preservatives were not used in the abovementioned pellet for conveying therapeutic substances.

Furthermore, advantageously, the production process is implemented by means of food grade systems for human feeding.

During the process, the pellet subject of the present invention does not exceed the temperature of 25 C, not even due to the friction force exerted during the compression.

## Claims

1. Product for feeding animals **characterised in that** it comprises:
- a package;
- pellets for feeding purposes contained in said package and externally coated with fat or sprayed with appetising substances; and
- a percentage in the range of 5 - 15 % of pellets for therapeutic purposes contained in said package and comprising a percentage of moisture lesser than 6% in a mixture of powder ingredients compacted by means of dry-pressing in form of pellets, said ingredients comprising: at least one thickening component for compacting the ingredients together by means of cold and dry pressing; at least one hydrophilic mineral for absorbing the moisture from the other components of the tablet and facilitating the separation of the mixture compacted in form of pellet from the press; at least one therapeutic substance for curing animals, containing thermolabile or oxidising active ingredients; said pellets for therapeutic purposes having lipophilic
characteristics so that they absorb fat appetising substances by simple contact with said pellets for feeding purposes so that part of the appetising substances are transferred onto the surface of the pellets for therapeutic purposes making also the latter quite appetising; the density of said pellet for therapeutic purposes being similar to the one of the pellet for feeding purposes to guarantee its uniformity of distribution inside the mass of the same pellets for feeding purposes,
**characterized in that** said mixture of powder ingredients of said pellets for therapeutic purposes also comprises carob flour in a quantity comprised in the range of 5-25 %.

2. Product for feeding animals according to claim 1, wherein the therapeutic substance is selected separately or combined in the family of products comprising: vitamin C, lysozyme, bioflavonoids, shark cartilage, contoutina, glucosamine, propolis, Echinacea, Astaxanthin, Papaya, Aloe, Ginseng, Pineapple, Rosa Canina, Resveratrol, Carnetin, Resveratrol, and other mineral, vegetable, animal substances with medicinal effects sensitive to heat and/or oxidant agents.

3. Product for feeding animals according to claim 1, wherein comprised among the thickening substances is carob flour.

4. Product for feeding animals according to claim 1, wherein comprised among the thickening substances is one or more of the following components: calcium dibasic phosphate, maize starch, maltodestrine.

5. Product for feeding animals according to claim 1, wherein among the hydrophilic minerals is magnesium stearate or silica.

6. Product for feeding animals according to claim 1, wherein the pellets for therapeutic purposes comprise carob flour as a thickening substance; the pellets for therapeutic purposes having a particularly dark colour, in particular brown, due to the carob flour contained therein, which is mixed with the other components and which has absorbed fats by capillarity upon contact with the pellets for feeding purposes.

7. Product for feeding animals according to claim 1, wherein the pellet for therapeutic purposes has a shape provided with corners, preventing its easy movement inside a mass of pellets for feeding purposes.

8. Process for producing a product for animal wherein:
- pellets for therapeutic purposes are mixed with pellets for feeding purposes externally coated with fat or sprayed with appetising substances;
- said pellets for therapeutic purposes are obtained by means of the following operation steps:
- providing for:
at least one thickening ingredient in form of powder,
at least one hydrophilic mineral in form of powder;
at least one therapeutic substance in form of powder for curing animals, containing thermolabile or oxidising active ingredients;
- at least one mixing step intended to uniform the ingredients together,
- one step for dry pressing said mixed powders to obtain compact agglomerations in form of pellets;
- said pellets for therapeutic purposes are mixed in percentage in the range of 5 to 15% with pellets for feeding purposes with a similar density for a uniform distribution therein;
- said pellets for therapeutic purposes having lipophilic
characteristics so that
through said mixing step, a step for absorbing part of the surface fats of said pellets for feeding purposes is performed by said pellets for therapeutic purposes; **characterized in that** said step of providing is also for a carob flour in a quantity comprised in the range of 5-25 %.

9. Process for producing a product for animal according to claim 8, wherein said pellets for feeding purposes are obtained by means of extrusion starting from a mixture of components introduced into an extrusion chamber and forced by the action of at least one worm screw, passing through the holes of a die arranged at the head of the extruder, said mixture exiting from the die is cut by blades in the desired form of pellets.

10. Process for producing a product for animal according to claim 9, wherein the extruded granules are subjected to fat coating and/or spraying processes using products of animal or plant origin.

## Patentansprüche

1. Pellet für Tiere, **dadurch gekennzeichnet, dass** es Folgendes umfasst:
- Verpackung;
- in der genannten Verpackung enthaltene und außen mit Fett beschichtete oder mit appetitanregenden Substanzen besprühte Pellets zu Futterzwecken; und
- einen Anteil von 5 - 15 % an in der genannten Verpackung enthaltenen Pellets für Therapiezwecke, die einen Feuchtigkeitsanteil von weniger als 6 % in einer Mischung von mittels Trockenpressung in Form von Pellets verdichteten Pulverbestandteilen umfassen, wobei die genannten Bestandteile Folgendes umfassen: mindestens eine eindickende Komponente zum Verdichten der Bestandteile miteinander mittels kalter und trockener Pressung; mindestens ein hydrophiles Mineral zum Aufnehmen der Feuchtigkeit aus den anderen Komponenten der Tablette und leichteren Trennen der in Pelletform in der Presse verdichteten Mischung; mindestens eine therapeutische Substanz zur Behandlung von Tieren, die thermolabile oder oxidierende Wirkstoffe enthält;
wobei die genannten Pellets für Therapiezwecke lipophile Eigenschaften aufweisen, so dass sie durch den einfachen Kontakt mit den genannten Pellets zu Futterzwecken fette, appetitanregende Substanzen absorbieren, so dass ein Teil der appetitanregenden Substanzen auf die Oberfläche der Pellets für Therapiezwecke übertragen werden und so auch Letztere appetitanregender machen; wobei die Dichte der genannten Pellets für Therapiezwecke der den Pellets für Futterzwecke ähnelt, um die Gleichmäßigkeit der Verteilung im Inneren der Masse der Pellets für Futterzwecke zu garantieren,
**dadurch gekennzeichnet, dass** die genannte Mischung von Pulverbestandteilen der genannten Pellets für Therapiezwecke außerdem Johannisbrotkernmehl in einer Menge zwischen 5 - 25 % umfasst.

2. Pellet für Tiere nach Anspruch 1, bei dem die therapeutische Substanz getrennt von oder in Verbindung mit der Produktfamilie gewählt wird, die Folgendes umfasst: Vitamin C, Lysozyme, Bioflavonoide, Haifischknorpel, Contoutina, Glucosamin, Propolis, Echinacea, Astaxanthin, Papaya, Aloe, Ginseng, Ananas, Hagebutte, Resveratrol, Carnitin und andere pflanzlichen Mineralien und gegenüber Wärme und Oxidationsmitteln empfindliche tierische Substanzen mit medizinischer Wirkung.

3. Pellet für Tiere nach Anspruch 1, dessen eindickende Substanzen Johannisbrotkernmehl umfassen.

4. Pellet für Tiere nach Anspruch 1, dessen eindickende Substanzen eine oder mehrere der folgenden Substanzen umfassen: Dicalciumphosphat, Maisstärke, Maltodextrin.

5. Pellet für Tiere nach Anspruch 1, in dem sich unter den hydrophilen Mineralien Magnesiumstearat oder Silizium befinden.

6. Pellet für Tiere nach Anspruch 1, in dem die Pellets zu Therapiezwecken Johannisbrotkernmehl als verdickende Substanz enthalten; wobei die Pellets für Therapiezwecke aufgrund des darin enthaltenen Johannisbrotkernmehls, das mit den anderen Komponenten gemischt wird und Fett durch Kapillarität beim Kontakt mit den Pellets für Futterzwecke aufgenommen hat, eine besonders dunkle Farbe aufweisen, insbesondere Braun.

7. Pellet für Tiere nach Anspruch 1, in dem die Pellets für Therapiezwecke eine eckige Form haben, die eine zu leichte Beweglichkeit in einer Masse von Pellets zu Futterzwecken verhindert.

8. Verfahren zur Herstellung eines für Tiere bestimmten Produkts, in dem:
- zu Therapiezwecken bestimmte Pellets mit außen mit Fett beschichteten oder mit appetitanregenden Substanzen besprühten, zu Futterzwecken bestimmten Pellets gemischt sind;
- die genannten Pellets zu Therapiezwecken anhand der folgenden Schritte erhalten werden:
- indem für Folgendes gesorgt wird:
mindestens ein verdickender Bestandteil in Pulverform;
mindestens ein hydrophiles Mineral in Pulverform;
mindestens eine therapeutische Substanz in Pulverform zur Behandlung von Tieren, die thermolabile oder oxidierende aktive Wirkstoffe enthält;
- mindestens einen Mischschritt zur gleichmäßigen Mischung der Bestandteile miteinander;
- einen Schritt zum Trockenpressen der genannten gemischten Pulver, um eine kompakte Agglomerierung in Pelletform zu erhalten;
- die genannten Pellets zu Therapiezwecken für eine gleichmäßige Verteilung zum einem Anteil von 5 bis 15 % mit Pellets zu Futterzwecken mit einer ähnlichen Dichte gemischt werden;
- wobei die genannten Pellets für Therapiezwecke lipophile Eigenschaften aufweisen, so dass durch den genannten Mischschritt ein Schritt zum Absorbieren eines Teils der Oberflächenfette der genannten Pellets zu Futterzwecken durch die genannten Pellets für Therapiezwecke erfolgt; **dadurch gekennzeichnet, dass** der genannte Schritt auch Johannisbrotkernmehl in einer Menge zwischen 5 - 25 % zuführt.

9. Verfahren zur Herstellung eines Produkts für Tiere nach Anspruch 8, bei dem die genannten Pellets für Futterzwecke mittels Extrusion ausgehend von einer Mischung von in eine Extrusionskammer eingeleiteten und mit Wirkung mindestens einer Schneckenschraube durch die Öffnungen eines Gesenks auf dem Extruderkopf gepressten Komponenten erzielt werden, wobei die aus dem Gesenk austretende Mischung durch Messer in die gewünschte Pelletform geschnitten wird.

10. Verfahren zur Herstellung eines Produkts für Tiere nach Anspruch 9, bei dem die extrudierten Granulate unter Verwendung von Produkten tierischen oder pflanzlichen Ursprung einer Fettbeschichtung bzw. einem Sprühverfahren unterzogen werden.

## Revendications

1. Granulés pour animaux **caractérisés en ce qu'**ils comprennent :
- un emballage ;
- granulés à des fins d'alimentation contenus dans ledit emballage revêtus extérieurement avec de la graisse ou traités avec des substances appétissantes ; et
- un pourcentage dans la plage de 5 - 15 % de granulés à des fins thérapeutiques contenus dans ledit emballage et comprenant un pourcentage d'humidité inférieur à 6 % dans un mélange d'ingrédients en poudre compactés au moyen d'un pressage à sec sous forme de granulés, lesdits ingrédients comprenant : au moins un composant épaississant pour compacter les ingrédients ensemble au moyen d'un pressage à froid et à sec ; au moins un minéral hydrophile pour absorber l'humidité des autres composants du comprimé et faciliter la séparation du mélange compacté sous forme de granulés par la presse ; au moins une substance thérapeutique pour soigner les animaux, contenant des ingrédients actifs thermolabiles ou oxydants ;
lesdits granulés à des fins thérapeutiques possédant des caractéristiques lipophiles de sorte qu'ils absorbent les substances graisses appétissantes par simple contact avec lesdits granulés à des fins d'alimentation de sorte qu'une partie des substances appétissantes soit transférée sur la surface des granulés à des fins thérapeutiques en rendant ces derniers appétissants ; la densité desdits granulés à des fins thérapeutiques étant similaire à celle des granulés à des fins d'alimentation pour garantir leur uniformité de distribution à l'intérieur de la masse des mêmes granulés à des fins d'alimentation,
**caractérisés en ce que** ledit mélange d'ingrédients en poudre desdits granulés à des fins thérapeutiques comprend également de la farine de caroube en quantité comprise dans la plage de 5 - 25 %.

2. Granulés pour animaux selon la revendication 1, dans lesquels la substance thérapeutique est sélectionnée séparément ou combinée dans une famille de produits comprenant : vitamine C, lysozyme, bio-flavonoïdes, cartilage de requin, contoutina, glucosamine, propolis, échinacée, astaxanthine, papaye, aloès, ginseng, ananas, rosier sauvage, resvératrol, carnitine, resvératrol, et d'autres minéraux, végétaux, substances animales avec des effets médicinaux sensibles à la chaleur et/ou agents oxydants.

3. Granulés pour animaux selon la revendication 1, dans lesquels la farine de caroube est comprise parmi les substances épaississantes.

4. Granulés pour animaux selon la revendication 1, dans lesquels parmi les substances épaississantes il y a un ou plusieurs des composants suivants : phosphate de calcium dibasique, amidon de maïs, maltodextrine.

5. Granulés pour animaux selon la revendication 1, dans lesquels parmi les minéraux hydrophiles il y a le stéarate de magnésium ou la silice.

6. Granulés pour animaux selon la revendication 1, dans lesquels les granulés à des fins thérapeutiques comprennent la farine de caroube en tant que substance épaississante; les granulés à des fins thérapeutiques ayant notamment une couleur foncée, brune en particulier, due à la farine de caroube qu'ils contiennent, qui est mélangée avec les autres composants et qui a absorbée la graisse par capillarité lors du contact avec les granulés à des fins d'alimentation.

7. Granulés pour animaux selon la revendication 1, dans lesquels les granulés à des fins thérapeutiques ont une forme avec des angles qui entravent leur libre mouvement à l'intérieur de la masse de granulés à des fins d'alimentation.

8. Procédé de fabrication de granulés pour animaux dans lequel :
- les granulés à des fins thérapeutiques sont mélangés avec les granulés à des fins d'alimentation revêtus extérieurement avec de la graisse ou traités avec des substances appétissantes ;
- lesdits granulés à des fins thérapeutiques sont obtenus à travers les étapes de fabrication suivantes :
- approvisionnant :
au moins un ingrédient épaississant sous forme de poudre ;
au moins un minéral hydrophile sous forme de poudre ;
au moins une substance thérapeutique sous forme de poudre pour soigner les animaux, contenant des ingrédients actifs thermolabiles ou oxydants ;
- au moins une étape de mélange pour homogénéiser les ingrédients ensemble ;
- une étape pour le pressage à froid desdites poudres mélangées pour obtenir une agglomération compacte sous forme de granulés ;
- lesdits granulés à des fins thérapeutiques sont mélangés en pourcentage dans la plage de 5 à 15 % avec les granulés à des fins d'alimentation ayant une densité similaire pour une distribution uniforme à l'intérieur de ceux-ci ;
- lesdits granulés à des fins thérapeutiques ayant des caractéristiques lipophiles de sorte qu'à travers ladite étape de mélange, une étape pour l'absorption d'une partie des graisses qui revêtent la surface desdits granulés à des fins d'alimentation est effectuée par lesdits granulés à des fins thérapeutiques ;
**caractérisé en ce que** ladite étape d'approvisionnement concerne aussi la farine de caroube en une quantité comprise dans la plage de 5 - 25 %.

9. Procédé de fabrication de granulés pour animaux selon la revendication 8, dans lequel lesdits granulés à des fins d'alimentation sont obtenus par extrusion à partir d'un mélange de composants introduits dans une chambre d'extrusion et forcés par l'action d'au moins une vis sans fin, à passer à travers les trous d'une filière montée sur la tête de l'extrudeuse, ledit mélange sortant de la filière étant coupé par des lames dans la forme de granulés souhaités.

10. Procédé de fabrication de granulés pour animaux selon la revendication 9 dans lequel les granulés extrudés sont soumis au procédé de revêtement de graisse et de traitement avec des produits d'origine animale ou végétale.
